(19) Europäisches Patentamt
European Patent Office
Office européen des brevets

(11) **EP 1 449 826 B1**

(12) **EUROPÄISCHE PATENTSCHRIFT**

(45) Veröffentlichungstag und Bekanntmachung des Hinweises auf die Patenterteilung:
**17.12.2008 Patentblatt 2008/51**

(51) Int Cl.:
***C07C 263/10*** *(2006.01)*  ***C07C 265/14*** *(2006.01)*
***B01J 10/00*** *(2006.01)*

(21) Anmeldenummer: **04002755.9**

(22) Anmeldetag: **07.02.2004**

(54) **Verfahren zur Herstellung von (Poly-)isocyanaten in der Gasphase**

Process for the production of (poly)isocyanates in the gas phase

Procédé de préparation de (poly)isocyanates en phase gazeuse

(84) Benannte Vertragsstaaten:
**AT BE BG CH CY CZ DE DK EE ES FI FR GB GR HU IE IT LI LU MC NL PT RO SE SI SK TR**

(30) Priorität: **20.02.2003 DE 10307141**

(43) Veröffentlichungstag der Anmeldung:
**25.08.2004 Patentblatt 2004/35**

(73) Patentinhaber: **Bayer MaterialScience AG**
**51368 Leverkusen (DE)**

(72) Erfinder:
• **Biskup, Klaus, Dr.**
**51373 Leverkusen (DE)**
• **Keldenich, Peter**
**40764 Langenfeld (DE)**
• **Fuhrmann, Peter**
**50769 Köln (DE)**
• **Six, Christian, Dr.**
**41468 Neuss (DE)**

(56) Entgegenhaltungen:
DD-A- 300 168          GB-A- 2 036 586
US-A- 4 847 408        US-A- 4 851 571
US-A- 5 449 818        US-A- 5 516 935
US-A- 5 931 579

EP 1 449 826 B1

**Beschreibung**

Die vorliegende Erfindung betrifft ein kontinuierliches Verfahren zur Herstellung von (Poly-)isocyanaten durch Phosgenierung der entsprechenden (Poly-)amine in der Gasphase mit optimierter Vermischung der Edukte.

Unter (Poly-)isocyanaten und (Poly-)amine werden dabei Mono-, Di und Polyisocyanate bzw. - amine verstanden.

Es ist bekannt, dass bei Gasphasenreaktionen die gute Vermischung der Edukte eine wichtige Rolle bei der Erzielung hoher Umsätze und Selektivitäten spielt, vor allem bei der Umsetzung polyfunktioneller Reaktanden. Eine optimale und nahezu spontane Mischung von Edukten ist entscheidend für die Wirtschaftlichkeit großtechnischer kontinuierlicher Verfahren, wenn

a) die Reaktion der Edukte nahezu spontan (hohe Reaktionsgeschwindigkeit),

b) ein oder mehrere Edukte mit dem Produkt bei vergleichbar hohen Reaktionsgeschwindigkeiten zu unerwünschten di- bzw. oligomeren Folgeprodukten reagieren,

c) die di- bis oligomeren Folgeprodukte einen deutlich höheren Siedepunkt als die Edukte bzw. das gewünschte Produkt aufweisen, bei Reaktionstemperatur im Reaktor kondensieren und an der Reaktorwandung Ablagerungen (z.B. Crackprodukte, polymere Folgeprodukte) bilden.

Beispielhaft sind die Gasphasenphosgenierung von aromatischen oder (cyclo-)aliphatischen polyfunktionellen Aminen im Rohrreaktor zu nennen. Im kontinuierlichen Verfahren werden üblicherweise die Edukte gasförmig in einen Reaktor eingebracht, wie dies in verschiedenen Patentanmeldungen beschrieben ist (z. B. EP-A 699 657, EP-A 676 392, EP-A 593 334, EP-A 570 799, EP-A 289 840).

Die Reaktion von Phosgen mit (Poly-)amin zu (Poly-)isocyanat steht mit der Folgereaktion von (Poly-)amin und (Poly-)isocyanat zum entsprechenden Harnstoffoligomer in Konkurrenz. Die Harnstoffoligomere der Gasphasenphosgenierung kondensieren bei den üblichen Reaktionstemperaturen im Rohrreaktor an der Reaktorwand. Eine verbesserte Mischung der Edukte Phosgen und (Poly-)amin bei gleichzeitiger Vermeidung von Rückströmung durch Wirbel im Rohrreaktor erhöht die Selektivität der (Poly-)isocyanatbildung und reduziert die Harnstoffbildung. Dadurch lassen sich die Mengen an Kondensationsprodukt im Rohrreaktor verringern, die aufgrund ihrer Ablagerung an der Reaktorwand zu einer Verkleinerung des freien Rohrquerschnitts und zu allmählichem Druckanstieg im Reaktor führen und letztendlich die Standzeit des Verfahrens bestimmen.

Die Vermischung der Reaktionspartner soll innerhalb einer Zeit von bis zu 0,5 Sekunden bis zu einem Segregationsgrad von 10-3 erfolgen. Der Segregationsgrad ist ein Maß für die Unvollständigkeit der Vermischung (EP-A 570 799).

Die Methoden zur Realisierung kurzer Mischzeiten sind im Prinzip bekannt. Geeignet sind Mischaggregate mit dynamischen oder statischen Mischorganen. Bevorzugt werden statische Mischer eingesetzt. Für die Konstruktion statischer Mischorgane ist eine Reihe verschiedener Realisierungsmöglichkeiten denkbar, z. B. die Verwendung von aus der Verbrennungstechnik bekannten Düsen, Glattstrahldüsen oder Venturidüsen.

Nachteile vieler Konstruktionen sind hoher Druckverlust oder eine Anordnung, die zu nicht ausreichend schneller Vermischung oder zu Rückvermischung in der Mischzone oder im Reaktionsraum führt. Hoher Druckverlust im Mischorgan bedingt einen erhöhten Aufwand bei der Zuführung der gasförmigen Edukte. Höherer Druckverlust erfordert eine erhöhte Siedetemperatur, um einen ausreichenden Vordruck zu gewährleisten. Besonders bei Edukten mit reaktiven funktionellen Gruppen hat die erhöhte Siedetemperatur aber thermische Schädigung und damit verstärkte Bildung von Nebenprodukten (Ausbeute- / Selektivitätsverluste) zur Folge. Nicht ausreichend schnelle Vermischung oder Rückvermischung führen zudem zu erhöhter Verweilzeit eines Teils der Edukte und Produkte und in Folge dessen zu unerwünschten Parallel- oder Folgereaktionen. Ferner bedingt eine unzureichende Vermischung vor allem bei stark exothermen oder endothermen Reaktionen eine ungleichmäßige Temperaturverteilung im Reaktor. Derartige "hot spots" oder "cold spots" im Reaktor resultieren in einer verstärkten thermischen Zersetzung der Produkte oder unerwünschter verfrühter Kondensation der Produkte. Thermische Zersetzungsprodukte bilden festen Rückstand, der sich an der Reaktorwand ablagert. In diesem Fall ist es üblich, den Reaktor mit einem sogenannten Inliner (Reaktionsrohr) auszurüsten, der bei Verkrustung ausgewechselt werden kann, so dass die Reinigung des Reaktors erleichtert ist. Beispielsweise ist im Falle eines Reaktors in Form eines zylindrischen Rohrs ein einfaches zylindrisch-gewälztes Stahlblech geringer Dicke aus beständigem Werkstoff als Inliner geeignet.

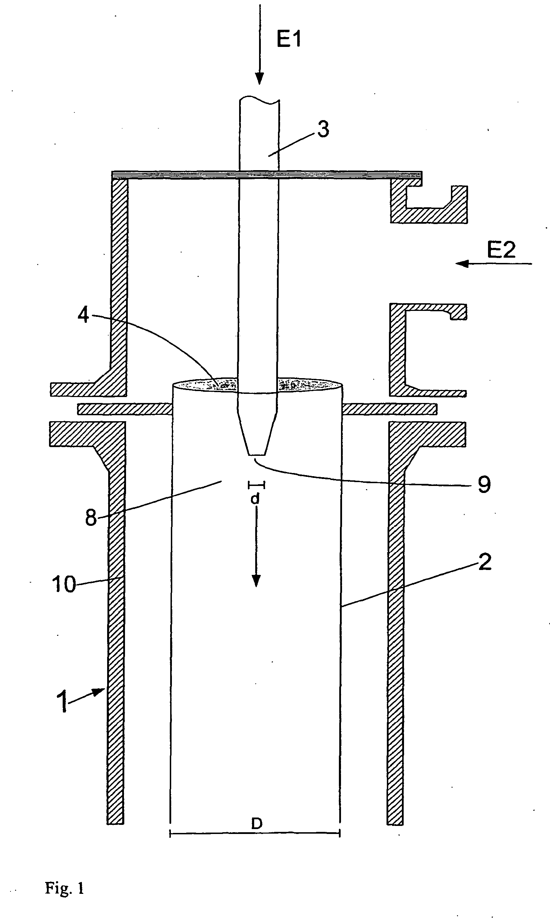

Die bekannten Nachteile können minimiert werden, wenn man als Mischorgan eine einfache einzelne Düse mit genau spezifizierten Maßen verwendet, die koaxial in ein Rohr eingebaut ist. Der Rohrreaktor weist dann also eine Zentraldüse und einen Ringraum zwischen der Zentraldüse und der Wand des Rohrreaktors auf. Die Düse mündet dabei unmittelbar in den Reaktionsraum (Figur 1). Die Vermischung der Edukte findet unmittelbar hinter dem Düsenaustritt statt. Ein gasförmiges Edukt E1 (Phosgen oder (Poly-)amin) wird dabei über die Zentraldüse, das andere gasförmige Edukt ((Poly-)amin oder Phosgen) über den Ringraum zwischen der Zentraldüse und der Wand des Rohrreaktos in den Reaktionsraum geführt. Auf diese Weise wird der Strom des Edukts E1 zentral in den Strom des Edukts E2 eingebracht und dort vermischt. Die Strömungsgeschwindigkeit von E1 muss dabei jedoch groß sein gegenüber der Strömungsgeschwindigkeit von E2. Allerdings ist die mit einer solchen Anordnung erzielbare Standzeit des Reaktors noch immer nicht gänzlich befriedigend.

Die Aufgabe der vorliegenden Erfindung war daher, ein Verfahren zur Phosgenierung von (Poly-)aminen zur Verfügung zu stellen, mit dem hohe Ausbeuten und gleichzeitig hohe Standzeiten des Reaktors erreicht werden können.

Es wurde gefunden, dass es für eine spezifische Geometrie von Mischorgan und Reaktor hinsichtlich der Standzeit des Verfahrens und der relativen Ausbeute von Vorteil ist, die freie Querschnittsfläche an der Austrittsöffnung der Einfachdüse des Edukts E1 auf mehrere in Summe flächengleiche Einzeldüsen (sogenannte Mehrfachdüse) aufzuteilen. Für die Gasphasenreaktion von (Poly-)aminen mit Phosgen zu (Poly-)isocyanaten im erfindungsgemäßen Verfahren hat sich ergeben, dass die Zuführung von (Poly-)amin beispielsweise über drei bis sechs ringförmig angeordneten Düsen in einen Phosgenstrom Vorteile gegenüber einer zur Gesamtfläche der Mehrfachdüse flächengleichen Einfachdüse (einzelne Düse) für das (Poly-)amin bietet.

Gegenstand der Erfindung ist ein Verfahren zur Herstellung von Diisocyanaten der allgemeinen Formel (I)

$$OCN - R - NCO \qquad (I),$$

in welcher

R    für einen (cyclo-)aliphatischen oder aromatischen Kohlenwasserstoffrest mit bis zu 15 Kohlenstoffatomen steht, mit der Maßgabe, dass zwischen beiden NCO-Gruppen mindestens 2 Kohlenstoffatome angeordnet sind,

durch Phosgenierung der entsprechenden Diamine der allgemeinen Formel (II)

$$H_2N - R - NH_2 \qquad (II),$$

in welcher

R    für einen (cyclo-)aliphatischen oder aromatischen Kohlenwasserstoffrest mit bis zu 15, vorzugsweise 4 bis 13 Kohlenstoffatomen steht, mit der Maßgabe, dass zwischen den beiden Aminogruppen mindestens zwei Kohlenstoffatome angeordnet sind,

bei dem die dampfförmigen Diamine, gegebenenfalls verdünnt mit einem Inertgas oder mit den Dämpfen eines inerten Lösungsmittels, und Phosgen getrennt auf Temperaturen von 200°C bis 600°C erhitzt und in einem Rohrreaktor vermischt und zur Reaktion gebracht werden, dadurch gekennzeichnet, dass in dem Rohrreaktor eine Anzahl n ≥2 von parallel zur Achse des Rohrreaktors ausgerichteten Düsen angeordnet sind, wobei der die Diamine enthaltende Strom dem Rohrreaktor über die n Düsen zugeführt wird und der Phosgenstrom dem Rohrreaktor über den verbleibenden freien Raum zugeführt wird.

Unter dampfförmigen Diaminen werden erfindungsgemäß Diamine verstanden, die gasförmig vorliegen und gegebenenfalls noch Anteile an unverdampften Tröpfchen an Diaminen (Aerosol) enthalten können. Bevorzugt enthalten die dampfförmigen Diamine jedoch keine Tröpfchen an unverdampften Diaminen.

Es können jedoch auch der die Diamine enthaltende Strom und der Phosgenstrom vertauscht werden, so dass der Phosgenstrom dem Rohrreaktor über die n Düsen zugeführt wird und der die Diamine enthaltende Strom dem Rohrreaktor über den verbleibenden freien Raum zugeführt wird.

Typische Beispiele geeigneter aliphatischer Diamine sind z.B. in der EP-A 0 289 840 genannt. Bevorzugt werden Isophorondiamin (IPDA), Hexamethylendiamin (HDA) und Bis(p-aminocyclohexyl)methan eingesetzt.

Typische Beispiele geeigneter aromatischer Diamine sind die reinen Isomeren oder die Isomerengemische des Diaminobenzols, des Diaminotoluols, des Diaminodimethylbenzols, des Diaminonaphthalins sowie des Diaminodiphenylmethans. Bevorzugt werden 2,4-/2,6-Toluylendiamin-Gemische der Isomerenverhältnisse 80/20 und 65/35 oder das reine 2,4-Toluylendiamin-Isomere eingesetzt.

Die Ausgangsamine der Formel (II) werden vor der Durchführung des erfindungsgemäßen Verfahrens verdampft und auf 200°C bis 600°C, vorzugsweise 250°C bis 450°C erhitzt und gegebenenfalls verdünnt mit einem Inertgas wie $N_2$, He, Ar oder mit den Dämpfen eines inerten Lösungsmittels, z. B. aromatischen Kohlenwasserstoffe mit oder ohne Halogensubstitution, dem Reaktor zugeführt.

Das bei der Phosgenierung verwendete Phosgen wird vor Einspeisung in den Reaktor auf eine Temperatur von 200°C bis 600°C, vorzugsweise 250°C bis 450°C erhitzt.

Der die Diamine enthaltende Strom wird über eine Mehrfachdüse aus n Einzeldüsen bevorzugt gleichen Durchmessers $d_n$ zentral in den Phosgenstrom eingebracht, wobei die Strömungsgeschwindigkeit des die Diamine enthaltenden Stroms am Düsenaustritt groß ist gegenüber der Strömungsgeschwindigkeit des Phosgenstroms. Vorzugsweise ist die Geschwindigkeit des die Diamine enthaltenden Stroms mindestens um den Faktor 5 - 40 größer als die Geschwindigkeit des Phosgenstroms.

Die Anordnung der Mehrfachdüse kann vorzugsweise variieren zwischen n = 2 bis 9 ringförmig angeordneten baugleichen Einzeldüsen. Bevorzugt sind ringförmig angeordnete Düsen mit n = 3 bis 6 Einzeldüsen, besonders bevorzugt ist n = 6. Alternativ dazu sind beispielsweise auch Anordnungen von n-1 ringförmig angeordneten Düsen um eine zentral angeordnete Düse mit n = 4 bis 8 realisierbar. Bevorzugt sind Mehrfachdüsen mit 3 bis 7 ringförmig angeordneten Einzeldüsen und einer Zentraldüse, bevorzugt 5 oder 6 ringförmig angeordnete Einzeldüsen und eine Zentraldüse. Zur Optimierung der Durchmischung und zur Vermeidung von Rückströmungen kann sich dabei der Durchmesser der Zentraldüse vom Durchmesser der n ringförmig angeordneten Einzeldüsen unterscheiden. Die Anordnung der n Einzeldüsen der Mehrfachdüse auf einer Kreislinie ist dabei vorzugsweise so zu wählen, dass sich das Verhältnis der Kreisringfläche zwischen Kreislinie und Reaktoraußenwand zur Fläche des durch die Kreislinie definierten Kreises im Bereich von 0,5 bis 3 bewegt, besonders bevorzugt im Bereich von 1 bis 2,5.

Aus dem Durchmesser der Einzeldüsen lässt sich ein fiktiver Durchmesser $d_{fiktiv}$ gemäß der Formel

$$d_{fiktiv} = \sqrt{n \cdot d_n^2}$$

berechen, wobei $d_n$ den Durchmesser der n baugleichen Einzeldüsen bedeutet. Der fiktive Durchmesser $d_{fiktiv}$ liegt vorzugsweise im Bereich von 5% bis 45% des Durchmessers D des Rohrreaktors.

Bei der Durchführung des erfindungsgemäßen Verfahrens liegt im allgemeinen der Druck in den Zuleitungen für den Phosgenstrom und den die Diamine enthaltenden Strom zum Reaktionsraum bei 200 mbar bis 3000 mbar und am Ausgang des Reaktionsraumes bei 150 mbar bis 2000 mbar, wobei durch Aufrechterhaltung eines geeigneten Differenzdruckes eine Strömungsgeschwindigkeit innerhalb des Reaktionsraumes von mindestens 1 m/s, vorzugsweise mindestens 3 m/s und besonders bevorzugt 5 m/s bis 120 m/s gewährleistet wird. Unter diesen Voraussetzungen herrschen innerhalb des Reaktionsraumes im allgemeinen turbulente Strömungsverhältnisse vor.

Die Vorteile des erfindungsgemäßen Verfahrens sind:

(a) eine Verkürzung der Mischzeiten gegenüber einer Einfachdüse (einzelnen Düse) mit gleicher Querschnittsfläche und damit einher gehend eine Verkürzung der erforderlichen Verweilzeit im Reaktor (Investitionskostenvorteil),

(b) eine verringerte Nebenproduktbildung sowie verkürzte thermische Belastung des Produktes und damit einher gehend eine Erhöhung der relativen Ausbeute,

(c) die Vermeidung bzw. Verringerung von festen Ablagerungen an der Reaktorwand und damit einher gehend eine Verlängerung der Standzeit des Verfahrens.

Die Erfindung wird anhand der Figuren nachfolgend näher erläutert. Es zeigen:

Figur 1    eine schematische Darstellung eines Rohrreaktors mit einer Zentraldüse nach dem Stand der Technik

Figur 2    eine schematische Darstellung eines Rohrreaktors mit 6 auf einer Kreislinie angeordneten Einzeldüsen

Figur 3    eine schematische Darstellung des in Figur 2 dargestellten Rohrreaktors in Seitenansicht.

Figur 1 zeigt eine schematische Darstellung eines Reaktors 1. Der Reaktor 1 enthält ein Gehäuse 10 in dem der Rohrreaktor 2 fixiert ist. Der durch Verdampfung erzeugte gasförmige Diamine enthaltende Strom E1 wird durch die Zentraldüse 3 in den Reaktionsraum 8 des Rohrreaktors 2 geleitet. Der Phosgenstrom E2 wird über den Ringraum 4 in den Reaktionsraum 8 geleitet. Direkt hinter dem Austritt 9 aus der Zentraldüse 3 werden der die Diamine enthaltende Strom E1 und der Phosgenstrom E2 vermischt und reagieren zum gewünschten Diisocyanat und unerwünschten Nebenprodukten.

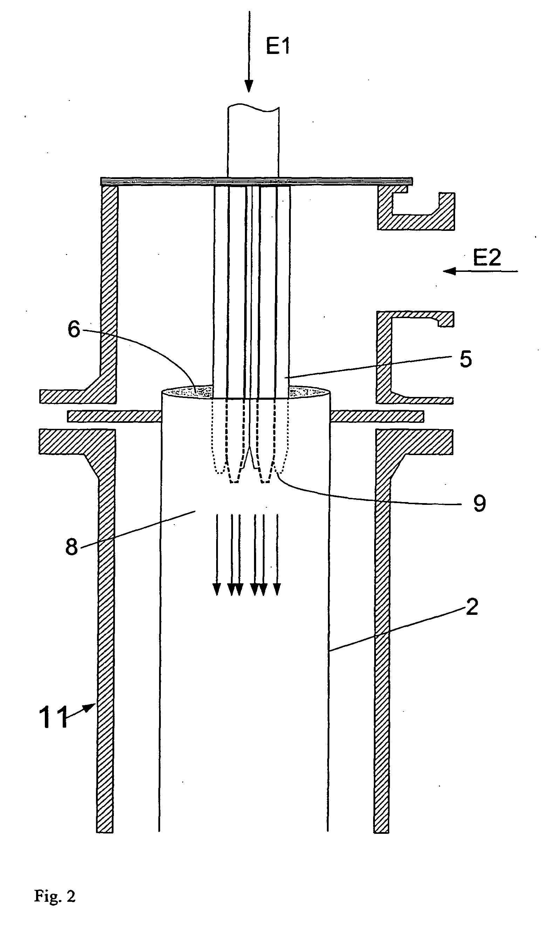

Figur 2 zeigt eine schematische Darstellung eines Reaktors 11, bei dem der gasförmige Diamine enthaltende Strom E1 auf n = 6 Einzeldüsen 5 aufgeteilt wird und aus den 6 Einzeldüsen 5 in den Reaktionsraum 8 strömt. Der Phosgenstrom E2 wird parallel zu den Düsen durch den verbleibenden freien Raum 6 in den Reaktionsraum 8 geleitet.

Der freie Raum 6 ist dabei der nicht von den Einzeldüsen 5 eingenommene Raum im Eingangsbereich des Rohrreaktors 2. Die Vermischung und Reaktion von Diamin und Phosgen erfolgt direkt hinter dem Austritt 9 des Diamins aus den Einzeldüsen 5.

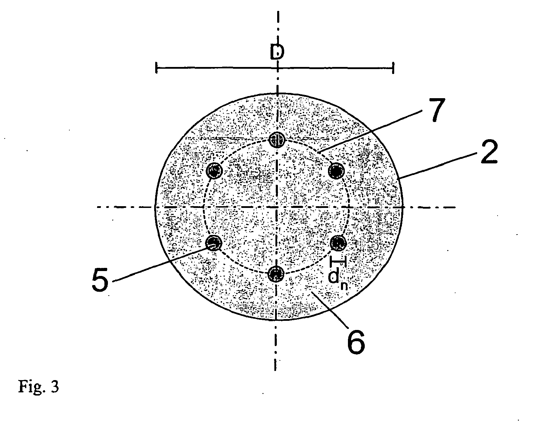

Figur 3 zeigt eine schematische Darstellung des Rohrreaktors 2 der Figur 2 in Frontalansicht. Die n = 6 Einzeldüsen 5 sind auf einer Kreislinie 7 angeordnet, wobei der Durchmesser jeder Einzeldüse $d_n$ und der Durchmesser des Rohrreaktors D beträgt. Der freie Raum 6 erstreckt sich dabei über die gesamte Querschnittsfläche des Rohrreaktors 2, befindet sich also sowohl zwischen der Kreislinie 7 und der Außenwand des Rohrreaktors 2 als auch innerhalb des durch die Kreislinie 7 definierten Raums.

**Beispiele**

Beispiel 1 (Vergleichsbeispiel)

In ein kombiniertes Misch- und Reaktionsrohr 2 gemäß Figur 1 mit nachgeschalteter Isocyanatkondensationsstufe und dieser nachfolgend eine Isocyanataufarbeitung (gemäß Stand der Technik) strömen kontinuierlich durch eine zentral angeordnete Einfachdüse 3, die in das Reaktionsrohr 2 hineinragt, ein Isomerengemisch von 2,4-/2,6-Toluylendiamin (TDA 80/20), Phosgen und Stickstoff in einem molaren Verhältnis von 1:6:0,2. Die Edukte werden getrennt voneinander in Reinform in vorgeschalteten Wärmetauschern verdampft und auf eine Temperatur von 340 - 370°C überhitzt. Ein Gemisch aus Stickstoff und TDA (E1) strömt durch die Einfachdüse 3, Phosgen (E2) im Ringraum 4 zwischen Rohrreaktor 2 und Einfachdüse 3. Das Verhältnis der Querschnittsflächen von Reaktionsraum zu Einfachdüse beträgt 192:1. Der Druck in der Reaktionszone liegt bei 1,5 bar. Die Strömungsgeschwindigkeit des Reaktionsgemisches hinter der Einfachdüse 3 im Reaktionsraum 8 beträgt ca. 1,6 m/s. Das Reaktionsprodukt 2,4-/2,6-Toluylendiisocyanat (TDI 80/20) wird nach einer Verweilzeit von ca. 2,5 sec in der Gasphase am Ende des Reaktionsrohres 2 durch Einspritzkühlung kondensiert, um im Anschluss die phosgenhaltige TDI-Rohware (Kondensat) zu entphosgenieren und destillativ aufzuarbeiten bzw. das Nebenprodukt Chlorwasserstoff (Brüden) zu reinigen. Die Ausbeutebestimmung an TDI erfolgt an Proben der phosgenhaltigen TDI-Rohware zu verschiedenen Zeiten des Versuches, gefolgt von destillativer Abtrennung und quantitativer Bestimmung polymerer Nebenprodukte sowie gaschromatographischer Bestimmung des TDI-Gehaltes im Destillat im Labor. Bezogen auf das eingesetzte TDA beträgt die TDI-Ausbeute max. 98,3 % d. Th. Die gewählte Anordnung erlaubt einen sehr gleichmäßigen Betrieb. Nach gezieltem Abfahren der Reaktion und Kontrolle des Reaktors auf Verschmutzungen nach 96 h Betriebsdauer sind im oberen Reaktionsteil ungleichmäßig verteilte Verschmutzungsbereiche auffällig, die bei länger fortdauerndem Betrieb strömungstechnisch nachteilige Auswirkungen haben und verstärkt zu Ablagerungen an der Wand des Reaktionsrohres 2 führen.

Beispiel 2 (erfindungsgemäß)

In ein kombiniertes Misch- und Reaktionsrohr 2 gemäß Figur 2 mit nachgeschalteter Isocyanatkondensationsstufe und dieser nachfolgend eine Isocyanataufarbeitung strömen kontinuierlich durch eine Mehrfachdüse bestehend aus 6 kreisförmig angeordneten Einzeldüsen 5, die in das Reaktionsrohr 2 hineinragt, ein Isomerengemisch von 2,4-/2,6-Toluylendiamin (TDA 80/20), Phosgen und Stickstoff in einem molaren Verhältnis von 1:6:0,2. Die Edukte werden getrennt voneinander in Reinform in vorgeschalteten Wärmetauschern verdampft und auf eine Temperatur von 340 - 370°C überhitzt. Ein Gemisch aus Stickstoff und TDA (E1) strömt durch die 6 kreisförmig angeordneten Einzeldüsen 5, Phosgen (E2) strömt im verbleibenden freien Raum 6 um die Einzeldüsen 5. Das Verhältnis der Querschnittsflächen von Reaktionsraum 8 zu Gesamtfläche der 6 Einzeldüsen 5 beträgt (analog Beispiel 1) 192:1. Der Druck in der Reaktionszone liegt bei 1,5 bar. Die Strömungsgeschwindigkeit des Reaktionsgemisches hinter der Mehrfachdüse beträgt ca. 1,6 m/s. Das Reaktionsprodukt 2,4-/2,6-Toluylendiisocyanat (TDI 80/20) wird nach einer Verweilzeit von ca. 2,5 sec in der Gasphase am Ende des Reaktionsrohres 2 durch Einspritzkühlung kondensiert, um im Anschluss die phosgenhaltige TDI-Rohware (Kondensat) zu entphosgenieren und destillativ aufzuarbeiten bzw. das Nebenprodukt Chlorwasserstoff (Brüden) zu reinigen. Die Ausbeutebestimmung an TDI erfolgt an Proben der phosgenhaltigen TDI-Rohware zu verschiedenen Zeiten des Versuches, gefolgt von destillativer Abtrennung und quantitativer Bestimmung polymerer Nebenprodukte sowie gaschromatographischer Bestimmung des TDI-Gehaltes im Destillat im Labor. Bezogen auf das eingesetzte TDA beträgt die TDI-Ausbeute max. 98,4 % d. Th. Die gewählte Anordnung erlaubt einen sehr gleichmäßigen Betrieb. Nach gezieltem Abfahren der Reaktion und Kontrolle des Reaktors auf Verschmutzungen nach 114 h Betriebsdauer sind im oberen Reaktionsteil nur geringfügige, gleichmäßig verteilte Verschmutzungsbereiche erkennbar, die bei länger fortdauerndem Betrieb keine strömungstechnischen Nachteile gebracht hätten.

**Patentansprüche**

1. Verfahren zur Herstellung von Diisocyanaten der allgemeinen Formel (I)

$$OCN - R - NCO \qquad (I),$$

in welcher

R für einen (cyclo-)aliphatischen oder aromatischen Kohlenwasserstoffrest mit bis zu 15 Kohlenstoffatomen steht, mit der Maßgabe, dass zwischen beiden NCO-Gruppen mindestens 2 Kohlenstoffatome angeordnet sind,

durch Phosgenierung der entsprechenden Diamine der allgemeinen Formel (II)

$$H_2N-R-NH_2 \qquad (II),$$

in welcher

R für einen (cyclo-)aliphatischen oder aromatischen Kohlenwasserstoffrest mit bis zu 15 Kohlenstoffatomen steht, mit der Maßgabe, dass zwischen den beiden Aminogruppen mindestens zwei Kohlenstoffatome angeordnet sind,

bei dem die dampfförmigen Diamine, gegebenenfalls verdünnt mit einem Inertgas oder mit den Dämpfen eines inerten Lösungsmittels, und Phosgen getrennt auf Temperaturen von 200°C bis 600°C erhitzt und in einem Rohrreaktor (2) vermischt und zur Reaktion gebracht werden, **dadurch gekennzeichnet, dass** in dem Rohrreaktor (2) eine Anzahl n ≥ 2 von parallel zur Achse des Rohrreaktors ausgerichteten Düsen (5) angeordnet sind, wobei der die Diamine enthaltende Strom dem Rohrreaktor (2) über die n Düsen (5) zugeführt wird und der Phosgenstrom dem Rohrreaktor (2) über den verbleibenden freien Raum (6) zugeführt wird.

2. Verfahren nach Anspruch 1, **dadurch gekennzeichnet, dass** der Phosgenstrom dem Rohrreaktor (2) über die n Düsen (5) zugeführt wird und der die Diamine enthaltende Strom dem Rohrreaktor (2) über den freien Raum (6) zugeführt wird.

3. Verfahren nach Anspruch 1, **dadurch gekennzeichnet, dass** Isophorondiamin (IPDA) oder Hexamethylendiamin (HDA) oder Bis(p-aminocyclohexyl)methan als Diamin der Formel (II) eingesetzt wird.

4. Verfahren nach Anspruch 1, **dadurch gekennzeichnet, dass** 2,4-/2,6-Toluylendiamin-Gemische als Diamin der Formel (II) eingesetzt wird.

5. Verfahren nach Anspruch 4, **dadurch gekennzeichnet, dass** 2,4-/2,6-Toluylendiamin-Gemische der Isomerenverhältnisse 80/20 und 65/35 oder das reine 2,4-Toluylendiamin-Isomere als Diamin der Formel (II) eingesetzt wird.

**Claims**

1. Process for the manufacture of diisocyanates of the general formula (I)

$$OCN - R - NCO \qquad (I),$$

in which

R represents a (cyclo-)aliphatic or aromatic hydrocarbon group having up to 15 carbon atoms, provided that at least 2 carbon atoms are arranged between the two NCO groups,

by phosgenation of the corresponding diamines of the general formula (II)

$$H_2N - R - NH_2 \qquad (II),$$

in which

R represents a (cyclo-)aliphatic or aromatic hydrocarbon group having up to 15 carbon atoms, provided that at least two carbon atoms are arranged between the two amino groups,

in which the vaporous diamine, optionally rarefied with an inert gas or with the vapours of an inert solvent, and phosgene are heated separately to temperatures of 200°C to 600°C and mixed and reacted in a tube reactor (2), **characterised in that** a number n ≥ 2 of nozzles (5) directed parallel to the axis of the tube reactor are arranged in the tube reactor (2), the diamine-containing stream being fed into the tube reactor (2) through the n nozzles (5) and the phosgene stream being fed into the tube reactor (2) through the remaining free space (6).

2. Process according to claim 1, **characterised in that** the phosgene stream is fed into the tube reactor (2) through the n nozzles (5) and the diamine-containing stream is fed into the tube reactor (2) through the free space (6).

3. Process according to claim 1, **characterised in that** isophorone diamine (IPDA) or hexamethylene diamine (HDA) or bis(p-aminocyclohexyl)-methane are used as the diamine of formula (II).

4. Process according to claim 1, **characterised in that** 2,4-/2,6-toluylene diamine mixtures are used as the diamine of formula (II).

5. Process according to claim 4, **characterised in that** 2,4-/2,6-toluylene diamine mixtures of isomer ratios 80/20 and 65/35 or pure 2,4-toluylene diamine isomers are used as the diamine of formula (II).

**Revendications**

1. Procédé pour la préparation de diisocyanates de la formule générale (I)

$$OCN - R - NCO \qquad (I),$$

dans laquelle

R représente un reste hydrocarboné (cyclo)aliphatique ou aromatique avec jusqu'à 15 atomes de carbone à condition qu'au moins deux atomes de carbone sont disposés entre les deux groupes NCO,

par phosgénation des diamines correspondantes de la formule générale (II)

$$H_2N - R - NH_2 \qquad (II),$$

dans laquelle

R représente un reste hydrocarboné (cyclo)aliphatique ou aromatique avec jusqu'à 15 atomes de carbone, à condition qu'au moins deux atomes de carbone sont disposés entre les deux groupes amino,

dans lequel les diamines sous forme de vapeur, éventuellement diluées avec un gaz inerte ou avec les vapeurs d'un solvant inerte, et du phosgène sont séparément chauffés à des températures de 200°C à 600°C et sont mélangés dans un réacteur tubulaire (2) et amenés à réagir, **caractérisé en ce qu'**il est disposé dans le réacteur tubulaire (2) un nombre n ≥ 2 de buses (5) orientées parallèlement à l'axe du réacteur tubulaire, le courant contenant les diamines étant introduit dans le réacteur tubulaire (2) par l'intermédiaire des n buses (5) et le courant de phosgène étant introduit dans le réacteur tubulaire (2) par l'intermédiaire de l'espace libre restant (6).

2. Procédé selon la revendication 1, **caractérisé en ce que** le courant de phosgène est introduit dans le réacteur tubulaire (2) par l'intermédiaire des n buses (5) et le courant contenant les diamines est introduit dans le réacteur tubulaire (2) par l'intermédiaire de l'espace libre (6).

3. Procédé selon la revendication 1, **caractérisé en ce que** l'on utilise de l'isophoronediamine (IPDA) ou de l'hexaméthylènediamine (HDA) ou du bis(p-aminocyclohexyl)méthane comme diamine de la formule (II).

4. Procédé selon la revendication 1, **caractérisé en ce que** l'on utilise des mélanges de 2,4-/2,6-toluylènediamine comme diamine de la formule (II).

**5.** Procédé selon la revendication 4, **caractérisé en ce que** l'on utilise des mélanges de 2,4-/2,6-toluylènediamine des rapports d'isomères 80/20 et 65/35 ou l'isomère de 2,4-toluylènediamine pur comme diamine de la formule (II).

Fig. 1

E1

E2

6

5

8

9

2

11

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 699657 A
- EP 676392 A
- EP 593334 A
- EP 570799 A
- EP 289840 A
- EP 0289840 A